(19) **Europäisches Patentamt / European Patent Office / Office européen des brevets**

(11) **EP 4 261 709 A1**

(12) # EUROPEAN PATENT APPLICATION
published in accordance with Art. 153(4) EPC

(43) Date of publication:
**18.10.2023 Bulletin 2023/42**

(21) Application number: **21902908.9**

(22) Date of filing: **19.02.2021**

(51) International Patent Classification (IPC):
**G06F 16/732** *(2019.01)*   **A61B 5/11** *(2006.01)*
**G06F 16/783** *(2019.01)*   **G06T 13/40** *(2011.01)*

(52) Cooperative Patent Classification (CPC):
**A61B 5/11; G06F 16/732; G06F 16/783;**
**G06T 13/40**

(86) International application number:
**PCT/JP2021/006290**

(87) International publication number:
**WO 2022/123800 (16.06.2022 Gazette 2022/24)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **08.12.2020 US 202063122509 P**

(71) Applicant: **Sony Group Corporation**
**Tokyo 108-0075 (JP)**

(72) Inventors:
• **MOCHIZUKI, Keita**
**Tokyo 108-0075 (JP)**
• **TANAKA, Yuki**
**Tokyo 108-0075 (JP)**

(74) Representative: **MFG Patentanwälte**
**Meyer-Wildhagen Meggle-Freund**
**Gerhard PartG mbB**
**Amalienstraße 62**
**80799 München (DE)**

(54) **INFORMATION PROCESSING DEVICE, INFORMATION PROCESSING METHOD, AND PROGRAM**

(57)     Provided are a novel and improved information processing method, information processing apparatus, and program that can improve user convenience.

The information processing apparatus includes an acquisition unit configured to acquire a processed feature amount that is a feature amount calculated by applying, to an unprocessed feature amount that is a feature amount of each time or each part of an object calculated from time-series data concerning motion of the object, a weight parameter prepared for each time or each part, and a search unit configured to search for motion data by using the processed feature amount acquired by the acquisition unit.

FIG.2

EP 4 261 709 A1

**Description**

[Technical Field]

The present disclosure relates to an information processing apparatus, an information processing method, and a program.

[Background Art]

In recent years, animation production and distribution using motion capture for acquiring motion information indicating the motion of a user have become increasingly popular. For example, motion data in which the motion of a user is mimicked is generated with use of acquired motion information, and avatar video based on the motion data in question is distributed.

Against such a background, the amount of motion data has been increasing year after year, and technologies for reusing previously generated motion data have accordingly been developed. For example, PTL 1 discloses a technology for concatenating multiple pieces of motion data to create animation data.

[Citation List]

[Patent Literature]

[PTL 1]
US Patent Application Publication No. 2012/0038628

[Summary]

[Technical Problems]

However, when users use motion data and animation data as described above, the users need to search for the motion data or the like by using text search or category search methods. As motion data increases in amount and becomes more complex, it may become difficult for users to search for motion data or the like that the users need.

Hence, the present disclosure proposes a novel and improved information processing method, information processing apparatus, and program that can improve user convenience.

[Solution to Problems]

According to the present disclosure, there is provided an information processing apparatus including an acquisition unit configured to acquire a processed feature amount that is a feature amount calculated by applying, to an unprocessed feature amount that is a feature amount of each time or each part of an object calculated from time-series data concerning motion of the object, a weight parameter prepared for each time or each part, and a search unit configured to search for motion data by using the processed feature amount acquired by the acquisition unit.

Further, according to the present disclosure, there is provided an information processing method that is executed by a computer, the information processing method including acquiring a processed feature amount that is a feature amount calculated by applying, to an unprocessed feature amount that is a feature amount of each time or each part of an object calculated from time-series data concerning motion of the object, a weight parameter prepared for each time or each part, and searching for motion data by using the processed feature amount acquired.

Further, according to the present disclosure, there is provided a program for causing a computer to achieve an acquisition function of acquiring a processed feature amount that is a feature amount calculated by applying, to an unprocessed feature amount that is a feature amount of each time or each part of an object calculated from time-series data concerning motion of the object, a weight parameter prepared for each time or each part, and a search function of searching for motion data by using the processed feature amount acquired by the acquisition function.

[Brief Description of Drawings]

[FIG. 1]

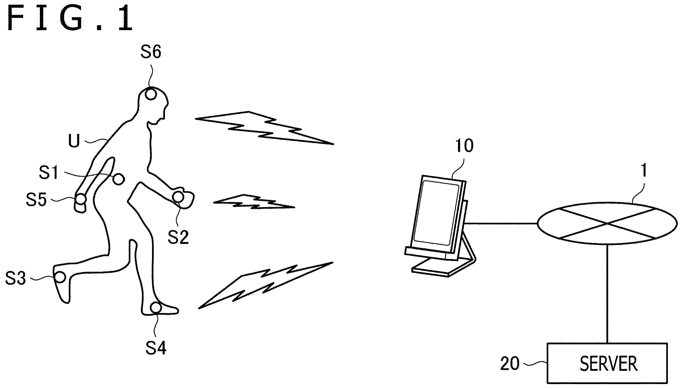

FIG. 1 is an explanatory diagram illustrating exemplary motion data search-related operation processing of an information processing terminal 10 according to the present disclosure.

[FIG. 2]

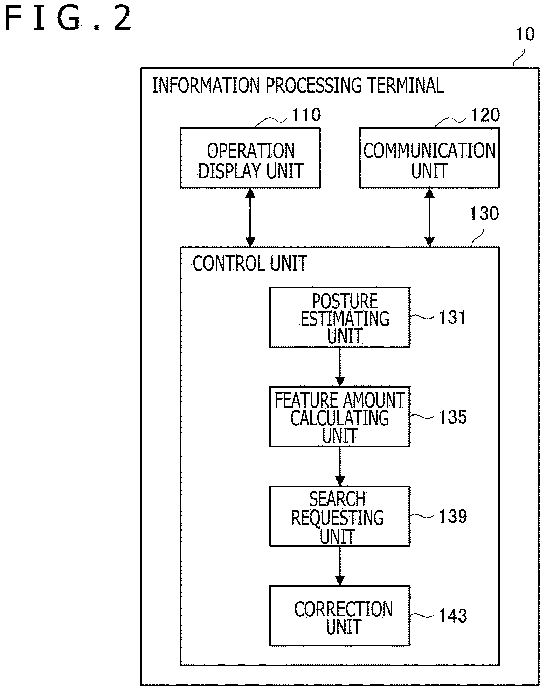

FIG. 2 is an explanatory diagram illustrating exemplary functional configurations of the information processing terminal 10 according to the present disclosure.

[FIG. 3]

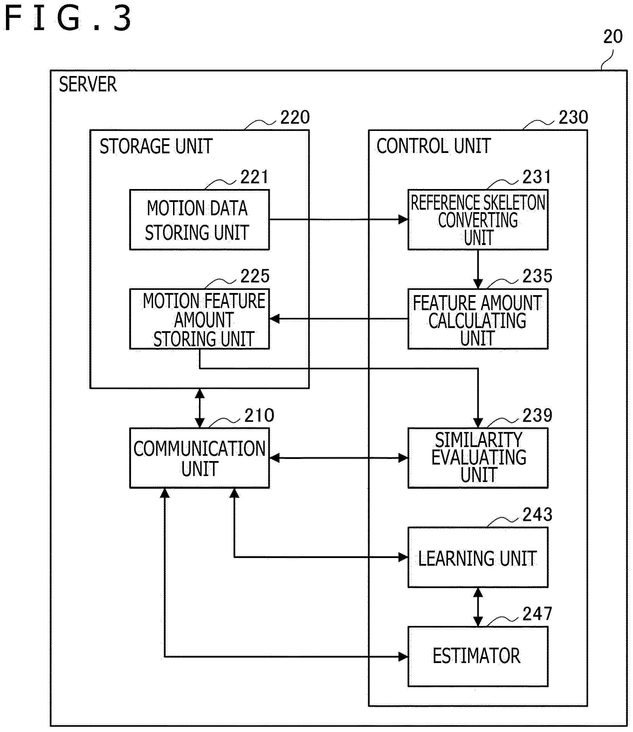

FIG. 3 is an explanatory diagram illustrating exemplary functional configurations of a server 20 according to the present disclosure.

[FIG. 4]

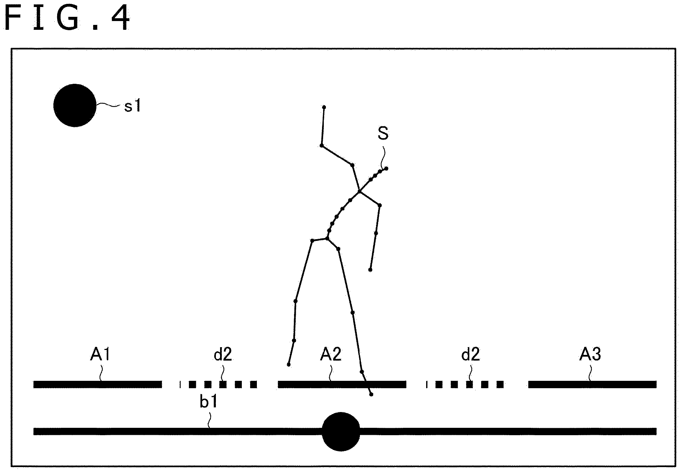

FIG. 4 is an explanatory diagram illustrating an exemplary GUI (Graphical User Interface) for concatenating multiple search results.

[FIG. 5]

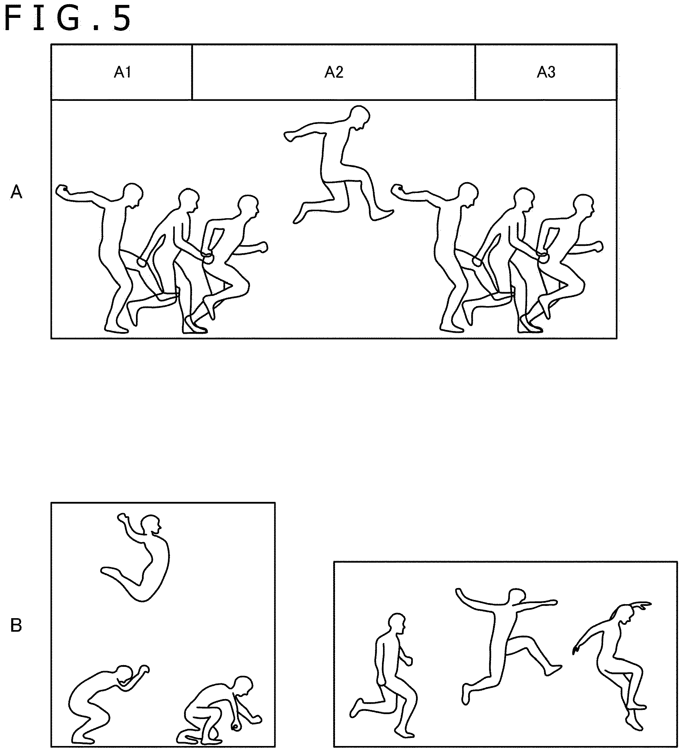

FIG. 5 depicts explanatory diagrams illustrating an example of modifying a section included in existing animation data to motion data.

[FIG. 6]

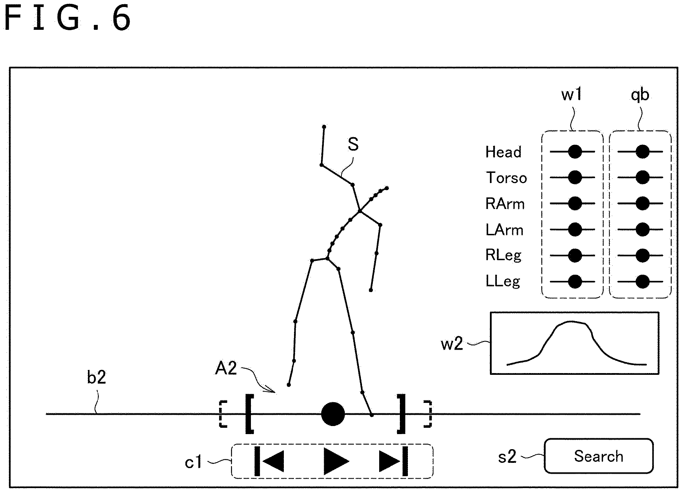

FIG. 6 is an explanatory diagram illustrating an exemplary GUI for modifying existing animations.

[FIG. 7]

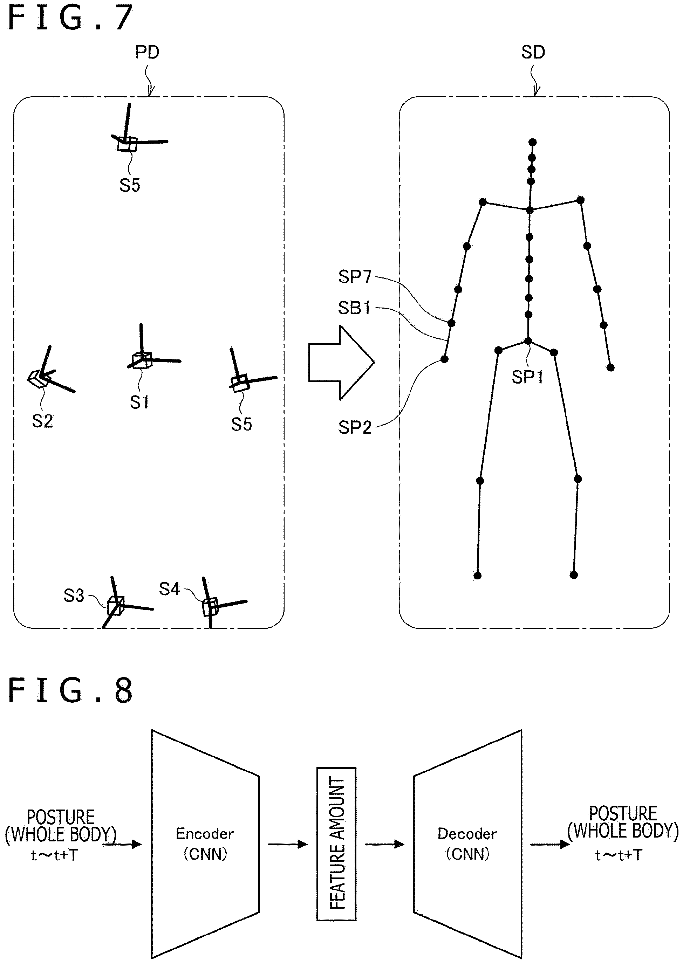

FIG. 7 is an explanatory diagram illustrating a specific example of a skeleton data generation method.

[FIG. 8]

FIG. 8 is an explanatory diagram illustrating an exemplary method of learning the relation between the time-series data of skeleton data and an unprocessed feature amount by using a machine learning technology.

[FIG. 9]

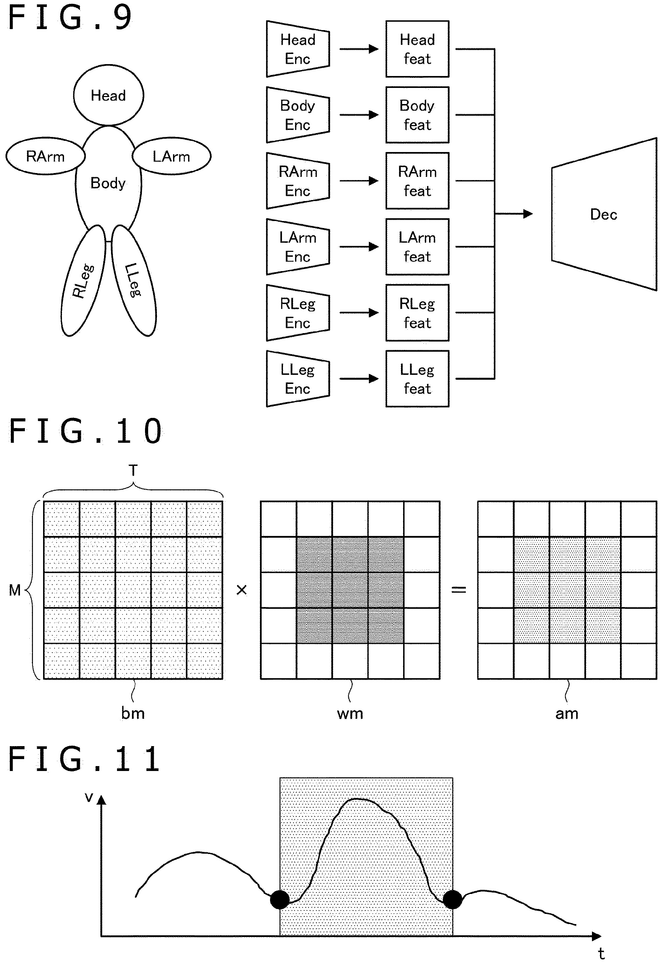

FIG. 9 is an explanatory diagram illustrating an exemplary method of calculating the unprocessed feature amount of each part according to the present disclosure.

[FIG. 10]

FIG. 10 is an explanatory diagram illustrating an exemplary method of calculating processed parameters by applying weight parameters to unprocessed feature amounts.

[FIG. 11]

FIG. 11 is an explanatory diagram illustrating an exemplary weight parameter prepared for each time.

[FIG. 12]

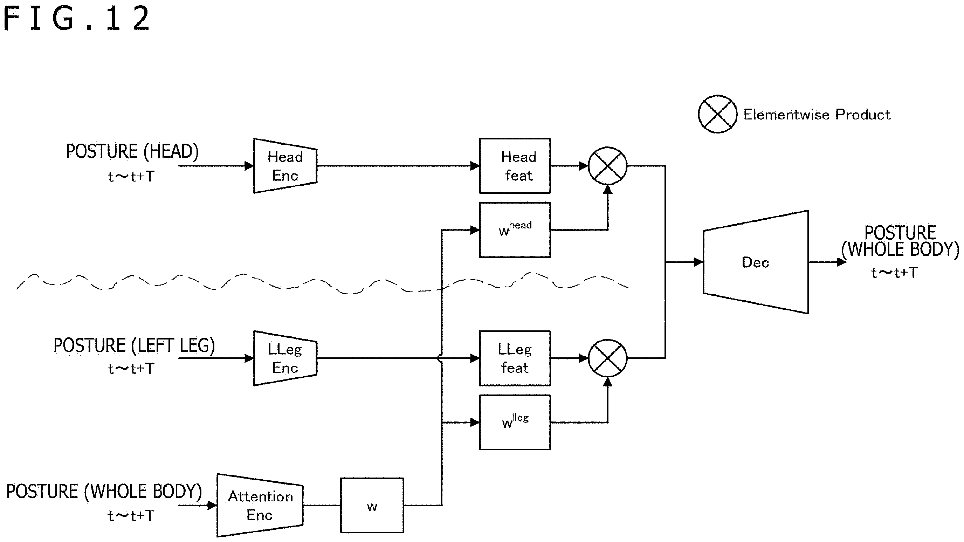

FIG. 12 is an explanatory diagram illustrating an exemplary weight parameter learning method.

[FIG. 13]

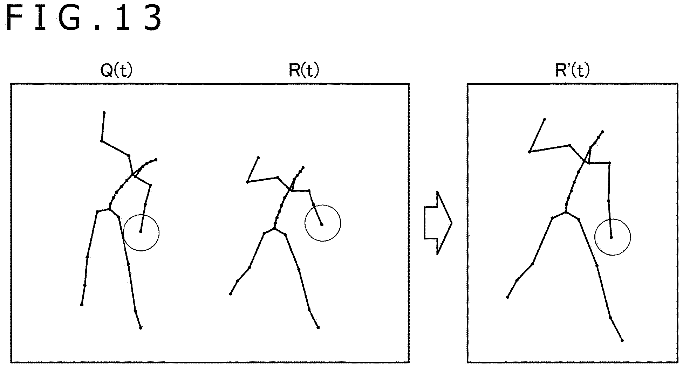

FIG. 13 is an explanatory diagram illustrating exemplary processing of correcting the feature amount of motion data.

[FIG. 14]

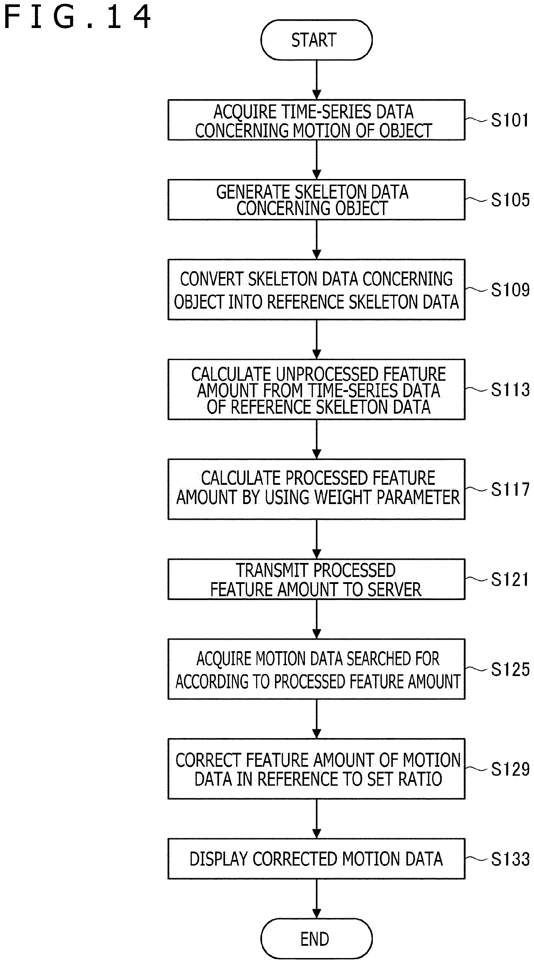

FIG. 14 is an explanatory diagram illustrating exemplary motion data search-related operation processing of the information processing terminal 10 according to the present disclosure.

[FIG. 15]

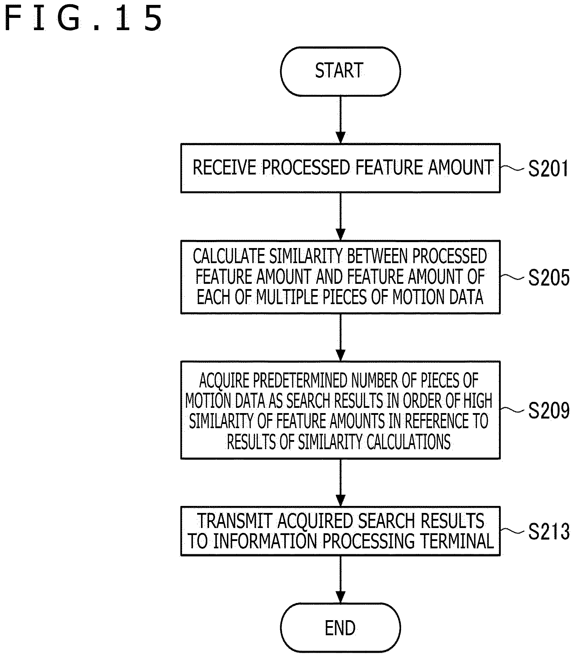

FIG. 15 is an explanatory diagram illustrating exemplary motion data search-related operation processing of the server 20 according to the present disclosure.

[FIG. 16]

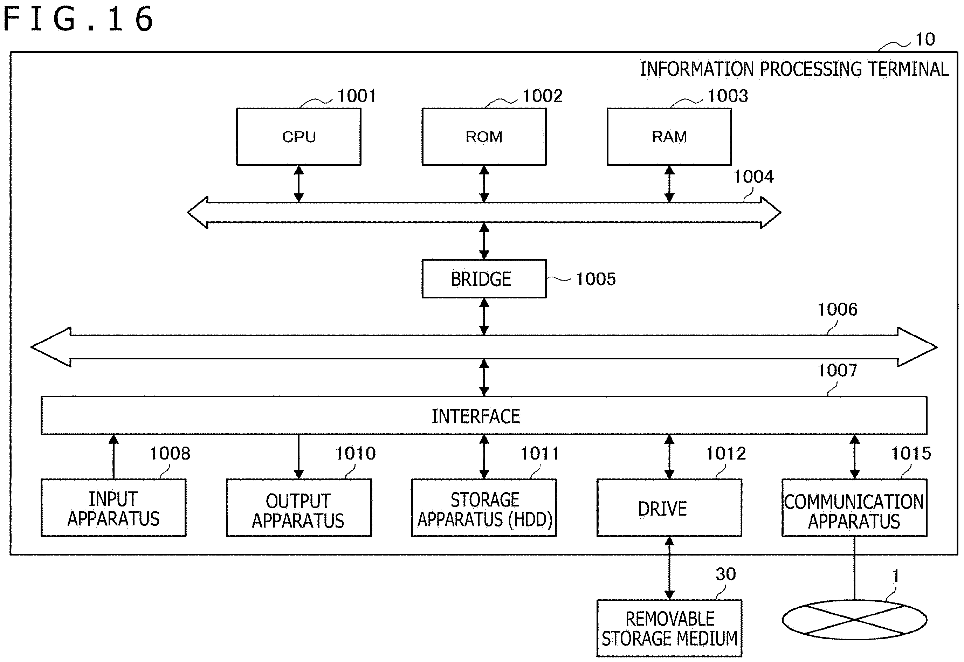

FIG. 16 is a block diagram illustrating the hardware configuration of the information processing terminal 10.

[Description of Embodiment]

A preferred embodiment of the present disclosure is described in detail below with reference to the accompanying drawings. Note that, in the present specification and the drawings, components that have substantially the same functional configurations are denoted by the same reference signs to omit redundant descriptions thereof.

Further, the items in the section "Description of Embodiment" are described in the following order.

1. Overview of Information Processing System

2. Exemplary Functional Configuration

    2-1. Exemplary Functional Configuration of Information Processing Terminal

    2-2. Exemplary Functional Configuration of Server

3. Details

    3-1. Specific Example of User Interface

    3-2. Posture Estimation

3-3. Feature Amount Calculation

3-4. Weight Parameter

3-5. Similarity Evaluation

3-6. Correction

4. Exemplary Operation

4-1. Operation of Information Processing Terminal

4-2. Operation of Server

5. Exemplary Action and Effect

6. Hardware Configuration

7. Supplementary Note

<<1. Overview of Information Processing System>>

As motion data, for example, skeleton data represented by a skeleton structure indicating the structure of a body is used to visualize information regarding the motion of a moving body such as a human or an animal. Skeleton data includes information regarding the positions or postures of parts. Note that, the parts of a skeleton structure correspond to the end parts or joint parts of a body, for example. Further, skeleton data may include bones that are line segments connecting parts to each other. The bones of a skeleton structure can correspond to human bones, for example; however, the positions and the number of bones may not be consistent with those of the actual human skeleton.

The position and posture of each part in skeleton data are acquirable by various motion capture technologies. For example, there are a camera-based technology in which markers are attached to respective parts of a body and the positions of the markers are acquired with use of an external camera or the like, and a sensor-based technology in which motion sensors are attached to parts of a body and position information regarding the motion sensors is acquired in reference to time-series data acquired by the motion sensors.

Further, the applications of skeleton data are diverse. For example, the time-series data of skeleton data is used for form improvement in sports, or is used for such applications as VR (Virtual Reality) or AR (Augmented Reality). Further, avatar video in which the motion of a user is mimicked is generated with use of the time-series data of skeleton data, and the avatar video in question is distributed.

In the following, as an embodiment of the present disclosure, an exemplary configuration of an information processing system configured to acquire the feature amount of skeleton data or the feature amount of each part in skeleton data calculated from time-series data concerning the motion of the whole body of a user and to search for motion data by using the feature amount in question is described. Note that, although humans are mainly described below as exemplary moving bodies, the embodiment of the present disclosure is also applicable to other moving bodies such as animals and robots.

FIG. 1 is an explanatory diagram illustrating the information processing system according to the embodiment of the present disclosure. As illustrated in FIG. 1, the information processing system according to the embodiment of the present disclosure includes six sensor apparatuses S1 to S6 that are attached to a user U, an information processing terminal 10, and a server 20.

The information processing terminal 10 is connected to the server 20 via a network 1. The network 1 is a wired or wireless transmission path for information transmitted from apparatuses connected to the network 1. Examples of the network 1 may include public networks such as the Internet, telephone networks, and satellite communication networks, various LANs (Local Area Networks) including Ethernet (registered trademark), WANs (Wide Area Networks), and dedicated line networks such as IP-VPNs (Internet Protocol-Virtual Private Networks).

(Sensor Apparatus S)

The sensor apparatus S detects the motion of the user U. The sensor apparatus S includes, for example, an inertial sensor (IMU: Inertial Measurement Unit) such as an acceleration sensor configured to acquire acceleration or a gyro sensor (angular velocity sensor) configured to acquire angular velocity.

Further, the sensor apparatus S may be any type of sensor apparatus equipped with sensors configured to detect the motion of the user U, such as an imaging sensor, a ToF (Time of Flight) sensor, a magnetic sensor, or an ultrasonic sensor.

The sensor apparatuses S1 to S6 are desirably attached to joint parts that serve as the references of the body (for example, waist or head) or to parts near the ends of the body (wrists, ankles, head, or the like). In the example illustrated in FIG. 1, the sensor apparatus S1 is attached to the waist of the user U, the sensor apparatuses S2 and S5 are attached to the respective wrists, the sensor apparatuses S3 and S4 are attached to the respective ankles, and the sensor apparatus S5 is attached to the head. Note that, in the following, a part of the body to which the sensor apparatus S is attached is sometimes also referred to as an "attachment part." Further, the number of the sensor apparatuses S and attachment positions (positions of attachment parts) are not limited to those in the example illustrated in FIG. 1, and the number of the sensor apparatuses S to be attached to the user U may be more or less.

Such a sensor apparatus S acquires the acceleration or angular velocity of an attachment part as time-series data and transmits the time-series data in question to the information processing terminal 10.

Further, the user U may not wear the sensor apparatus S. For example, the information processing terminal 10 may detect the motion of the user U by using various sensors (for example, an imaging sensor or a ToF sensor) included in the information processing terminal 10.

(Information Processing Terminal 10)

The information processing terminal 10 is an example of an information processing apparatus. The information processing terminal 10 calculates the feature amount of the motion of the user U from time-series data received from the sensor apparatus S and searches for motion data by using the calculated feature amount.

For example, the information processing terminal 10 transmits a processed feature amount as a search request to the server 20. Then, the information processing terminal 10 receives, from the server 20, motion data searched for by the server 20 in response to the search request in question.

Note that, although a smartphone is illustrated as the information processing terminal 10 in FIG. 1, the information processing terminal 10 may be another information processing apparatus such as a laptop PC (Personal Computer) or a desktop PC.

(Server 20)

The server 20 holds multiple pieces of motion data and the feature amount of each of the multiple pieces of motion data. Further, the server 20 evaluates the similarity between the feature amount of each of the multiple pieces of motion data and a processed feature amount received from the information processing terminal 10, and transmits motion data corresponding to the results of similarity evaluation to the information processing terminal 10.

In the above, the overview of the information processing system in the present disclosure is described. Next, exemplary functional configurations of the information processing terminal 10 and the server 20 according to the present disclosure are described.

<<2. Exemplary Functional Configuration>>

<2-1. Exemplary Functional Configuration of Information Processing Terminal>

FIG. 2 is an explanatory diagram illustrating exemplary functional configurations of the information processing terminal 10 according to the present disclosure. As illustrated in FIG. 2, the information processing terminal 10 includes an operation display unit 110, a communication unit 120, and a control unit 130.

(Operation Display Unit 110)

The operation display unit 110 has a function as a display unit configured to display search results transmitted from the server 20. Further, the operation display unit 110 has a function as an operation unit configured to allow the user to perform operation input.

The function as a display unit is achieved by, for example, a CRT (Cathode Ray Tube) display apparatus, a liquid crystal display (LCD) apparatus, or an OLED (Organic Light Emitting Diode) apparatus.

Further, the function as an operation unit is achieved by, for example, a touch panel, a keyboard, or a mouse.

Note that, although the information processing terminal 10 integrates the display unit function and the operation unit function in FIG. 1, the information processing terminal 10 may have the display unit function and the operation unit function separately.

(Communication Unit 120)

The communication unit 120 communicates various types of information with the server 20 via the network 1. For example, the communication unit 120 transmits skeleton data calculated from time-series data concerning the motion of the user and processed to the server 20. Further, the communication unit 120 receives motion data searched for by the server 20 according to a transmitted processed feature amount.

(Control Unit 130)

The control unit 130 controls the overall operation of the information processing terminal 10. As illustrated in FIG. 2, the control unit 130 includes a posture estimating unit 131, a feature amount calculating unit 135, a search requesting unit 139, and a correction unit 143.

The posture estimating unit 131 estimates attachment part information indicating the position and posture of each attachment part, in reference to time-series data such as the acceleration or velocity of the attachment part acquired from the sensor apparatus S. Note that, the position and posture of each attachment part may be a two-dimensional position or a three-dimensional position.

Further, the posture estimating unit 131 generates skeleton data including position information and posture information regarding each part of the skeleton structure, in reference to the attachment part information. Further, the posture estimating unit 131 may convert the generated skeleton data into reference skeleton data. Details regarding posture estimation are described later.

The feature amount calculating unit 135 is an example of an acquisition unit and calculates an unprocessed feature amount that is the feature amount of the whole body or the feature amount of each part of skeleton data from the time-series data of the skeleton data. Further, the feature amount calculating unit 135 calculates a processed feature amount by applying a weight parameter to the unprocessed feature amount. Details of unprocessed feature amounts, weight parameters, and processed feature amounts are described later.

The search requesting unit 139 is an example of a search unit and causes the communication unit 120 to transmit, as a search request, a processed feature amount calculated by the feature amount calculating unit 135.

The correction unit 143 corrects the feature amount of motion data by mixing a processed feature amount with the feature amount of motion data received as a search result from the server 20, at a set ratio. Details regarding correction are described later.

In the above, the exemplary functional configurations of the information processing terminal 10 have been described. Next, with reference to FIG. 3, the exemplary functional configurations of the server 20 are described.

<2-2. Exemplary Functional Configuration of Server>

FIG. 3 is an explanatory diagram illustrating exemplary functional configurations of the server 20 according to the present disclosure. As illustrated in FIG. 3, the server 20 includes a communication unit 210, a storage unit 220, and a control unit 230.

(Communication Unit 210)

The communication unit 210 communicates various types of information with the information processing terminal 10 via the network 1. For example, the communication unit 210 receives, from the information processing terminal 10, the processed feature amount of the whole body or each part in skeleton data calculated from time-series data concerning the motion of the user. Further, the communication unit 210 transmits, to the information processing terminal 10, motion data searched for according to a processed feature amount received from the information processing terminal 10.

(Storage Unit 220)

The storage unit 220 holds software and various types of data. As illustrated in FIG. 3, the storage unit 220 includes a motion data storing unit 221 and a motion feature amount storing unit 225.

The motion data storing unit 221 holds multiple pieces of motion data.

The motion feature amount storing unit 225 holds the feature amount of each of multiple pieces of motion data held by the motion data storing unit 221. More specifically, the motion feature amount storing unit 225 holds the feature amount of reference motion data that is motion data with the corresponding skeleton data converted into reference skeleton data.

(Control Unit 230)

The control unit 230 controls the overall operation of the server 20. As illustrated in FIG. 3, the control unit 230 includes a reference skeleton converting unit 231, a feature amount calculating unit 235, a similarity evaluating unit 239, a learning unit 243, and an estimator 247.

The reference skeleton converting unit 231 converts skeleton data included in each of multiple pieces of motion data into reference skeleton data. More specifically, the reference skeleton converting unit 231 converts the skeleton of each part included in each piece of skeleton data into a reference skeleton having corresponding predetermined skeleton information.

The feature amount calculating unit 235 calculates the feature amount of motion data converted into reference skeleton data and outputs the result of feature amount calculation to the motion feature amount storing unit 225. Note that, motion data converted into reference skeleton data is an example of reference motion data.

The similarity evaluating unit 239 evaluates the similarity between a processed feature amount received from the information processing terminal 10 and the feature amount of each of multiple pieces of motion data held by the motion feature amount storing unit 225. Details of similarity evaluation are described later.

The learning unit 243 generates learning data by a machine learning technology that uses, as supervised data, the combination of time-series data concerning each part in skeleton data and the feature amount of each part in motion data.

Further, the learning unit 243 may acquire the weight parameter for each part or the weight parameter for each time by using attention in a machine learning technology that uses, as supervised data, the combination of the time-series data of skeleton data and the feature amount of each part in motion data.

The estimator 247 estimates the unprocessed feature amount of each part from skeleton data concerning the user. The function of the estimator 247 is obtained from learning data generated by the learning unit 243.

In the above, the exemplary functional configurations according to the present disclosure have been described. Next, with reference to FIG. 4 to FIG. 13, details of the system according to the present disclosure are sequentially described.

<<3. Details>>

<3-1. Specific Example of User Interface>

The user performs operations on the display screen of the operation display unit 110 to search for motion data or modify existing animation data. In the present disclosure, as an example of searching for motion data, an example in which multiple pieces of motion data searched for according to the motion of the user are concatenated to generate a single piece of animation data is described. Further, as an example of modifying animation data, an example in which a section included in existing animation data is modified to motion data searched for according to a weight parameter is described.

(Concatenation of Search Results)

FIG. 4 is an explanatory diagram illustrating an exemplary GUI (Graphical User Interface) for concatenating multiple search results. The GUI for concatenating multiple search results may include skeleton data s, a search button s1, sections A1 to A3, a correction section d2, and a seek bar b1, as illustrated in FIG. 4.

The search button s1 is a button for turning ON or OFF a search function that acquires motion information regarding the user. Further, the sections A1 to A3 are sections into which motion data searched for according to the motion of the user is inserted, and the correction section d2 is a section that connects two sections into which motion data is inserted. Further, the seek bar b1 is an indicator bar for displaying the skeleton data s at the timing specified with a cursor.

The following operations and processing are performed on the GUI in question.

(1) First, the user selects the search button s1 according to a predetermined operation, to turn ON the search function.
(2) Next, the user performs an operation including information that the user wants to search for as motion data.
(3) Subsequently, the user selects the search button s1 again to turn OFF the search function.
(4) Then, the operation display unit 110 displays motion data searched for according to the motion of the user.
(5) In a case where multiple pieces of motion data searched for according to the motion of the user are displayed, the user selects one of the multiple pieces of displayed motion data.
(6) Further, the user selects any of the sections A1 to A3 as an insertion section.
(7) Then, the operation display unit 110 inserts the motion data into the section selected by the user.

The operations and processing of (1) to (7) are repeated multiple times to generate animation data in which multiple pieces of motion data are concatenated.

Note that, the correction section d2 is optional. The correction section d2 may be filled by use of any correction method, or animation data may be generated by multiple insertion sections being connected without the correction section d2.

Further, the operation display unit 110 may display the seek bar b1 to allow the user to check animation data generated by pieces of motion data being concatenated.

Further, in (6), the user may not specify an insertion section. For example, motion data may be inserted in order from sections earlier in time. For example, when the operations and processing of (1) to (5) are executed multiple times, motion data selected by the user in (5) may be inserted in order from the section A1. Further, the information processing terminal 10 may use any correction method in the correction sections d2 between the section A1 and the section A2 and between the section A2 and the section A3 to concatenate the pieces of motion data in the respective sections.

Further, although FIG. 4 illustrates the three sections A1 to A3 as sections into which motion data is inserted, the number of sections for insertion may not be three. Depending on the number of times the operations and processing of (1) to (5) are performed, the number of sections into which motion data is inserted may be determined.

Further, as will be described in detail later, the operation display unit 110 may display setting fields for various parameters, such as various weight parameters and set ratios for processed feature amounts and the feature amounts of motion data.

Next, with reference to FIG. 5 and FIG. 6, an example of modifying a section included in existing animation data to motion data is described.

(Modification of Existing Animation Data)

FIG. 5 depicts explanatory diagrams illustrating an example of modifying a section included in existing animation data to motion data. In the embodiment according to the present disclosure, a section included in animation data (hereinafter referred to as "existing animation data A") obtained by motion capture or manual work may be replaced with motion data B and modified.

For example, the user selects the section A2 as a modification section from among the multiple sections A1 to A3 included in existing animation data.

Then, the operation display unit 110 may display, in place of the section A2 in the existing animation data, the motion data B searched for according to the processed feature amount of the time-series data of the skeleton data included in the section A2.

For example, as illustrated in FIG. 5, in a case where two pieces of motion data B are displayed as search results, the user selects one of the pieces of motion data B. In a case where the user selects the left image of the motion data B illustrated in FIG. 5, the operation display unit 110 displays, in place of the section A2 in the existing animation, the left image of the motion data B illustrated in FIG. 5.

The example of modifying an existing animation according to the present disclosure is more specifically described with reference to FIG. 6.

FIG. 6 is an explanatory diagram illustrating an exemplary GUI for modifying existing animations. As illustrated in FIG. 6, the GUI for modifying existing animations may include the skeleton data s, a part-specific weight parameter setting field w1, a time-specific weight parameter setting field w2, a set ratio setting field qb, a search button s2, the section A2, a seek bar b2, and a reproduction command c1.

The part-specific weight parameter setting field w1 is a setting field for setting a weight parameter to be applied to an unprocessed feature amount calculated for each part. Further, the time-specific weight parameter setting field w2 is a setting field for setting a weight parameter to be applied to an unprocessed feature amount calculated for each time. Further, the set ratio setting field qb is a setting field for setting a ratio for mixing a processed feature amount with the feature amount of motion data concerning each part. Details of the weight parameter for each part, the weight parameter for each time, and set ratios are described later.

Further, the user can check modified animation data by operating the reproduction command c1. Note that, the user may check modified animation data by operating the seek bar b2.

First, the user selects the section A2 as a modification section. Subsequently, the user sets various parameters in the respective setting fields, i.e., the part-specific weight parameter setting field w1, the time-specific weight parameter setting field w2, and the set ratio setting field qb, and selects the search button s2.

Then, the operation display unit 110 displays at least one piece of motion data searched for in response to the operation performed by the user. In a case where a single piece of motion data is displayed as a search result, the operation display unit 110 inserts the motion data in question in place of the section A2. In a case where multiple pieces of motion data are displayed as search results, the user selects one of the multiple pieces of motion data, and the operation display unit 110 inserts the single piece of motion data selected by the user, in place of the section A2.

While the specific example of the user interface has been described above, the embodiment according to the present disclosure is not limited to this example. For example, when an existing animation is modified, the information processing terminal 10 may present modification candidate sections to the user, unlike in the described example in which the user selects a section to be modified. For example, the operation display unit 110 may present modification candidate sections to the user along with displaying existing animation data. In this case, the user may perform an operation to change the presented modification candidate sections.

Note that, a modification candidate section that is presented by the operation display unit 110 may be, for example, a section with relatively large motion among all sections in existing animation data or a section estimated to be particularly important with use of a machine learning technology such as a DNN (Deep Neural Network).

<3-2. Posture Estimation>

FIG. 7 is an explanatory diagram illustrating a specific example of a skeleton data generation method. The posture estimating unit 131 acquires, in reference to time-series data, attachment part information PD including position information and posture information regarding the attachment parts to which the sensor apparatuses S1 to S6 are attached, as illustrated in the left part of FIG. 7.

Moreover, the posture estimating unit 131 acquires, in reference to the attachment part information PD regarding the attachment parts, skeleton data SD including position information and posture information regarding each part in the skeleton structure, as illustrated in the right part of FIG. 7. The skeleton data SD includes not only information regarding an attachment part SP1 corresponding to the attachment part to which the sensor apparatus S1 is attached and an attachment part SP2 corresponding to the attachment part to which the sensor apparatus S2 is attached, but also information regarding a non-attachment part SP7.

Note that, the skeleton data SD can include information (position information, posture information, or the like) regarding bones in addition to part information. For example, in the example illustrated in FIG. 7, the skeleton data SD can include information regarding a bone SB1. The posture estimating unit can identify, in reference to position information and posture information regarding parts in a skeleton structure, information regarding a bone between the parts.

Further, the motion of the user may be detected with use of an imaging sensor or a ToF sensor included in the information processing terminal 10. In this case, the posture estimating unit 131 may generate the skeleton data SD concerning the user by using an estimator obtained by a machine learning technology that uses, as supervised data, the combination of time-series data concerning an image acquired by photographing a person and skeleton data.

Further, as will be described in detail later, when the similarity between a processed feature amount calculated from the time-series data of the skeleton data SD generated in reference to attachment part information and the feature amount of each of multiple pieces of motion data held by the motion data storing unit 221 is evaluated, it is sometimes better to convert the respective pieces of skeleton data into the same skeleton information (bone length, bone thickness, or the like) before evaluation.

As such, the posture estimating unit 131 may convert the skeleton of each part in the skeleton data SD into a reference skeleton to convert the skeleton data SD into reference skeleton data. However, in a case where similarity evaluation based on skeleton-independent feature amounts is performed, the posture estimating unit 131 may not convert the skeleton data SD into reference skeleton data. Examples of skeleton-independent feature amounts include posture information regarding each part.

The posture estimating unit 131 may convert the skeleton data SD into reference skeleton data by using any method, for example. Examples of any method include copying the posture of each joint, scaling a root position according to height, and adjusting the end position of each part by using IK (Inverse Kinematics).

Further, the learning unit 243 included in the server 20 may perform learning by using a DNN to separate the skeleton information and motion information of skeleton data. By using the estimator 247 obtained by learning, the posture estimating unit 131 may omit the processing of converting the skeleton data SD into reference skeleton data. In the following description, reference skeleton data is sometimes simply referred to as "skeleton data."

<3-3. Feature Amount Calculation>

In the present disclosure, feature amounts are divided into two types for description: unprocessed feature amounts and processed feature amounts obtained by applying weight parameters described later to unprocessed feature amounts.

The feature amount calculating unit 135 calculates an unprocessed feature amount from the time-series data of skeleton data estimated by the posture estimating unit 131.

For example, an unprocessed feature amount may be the velocity, position, or posture (rotation or the like) of each joint, or may be ground contact information.

Further, the learning unit 243 may learn the relation between the time-series data of skeleton data and an unprocessed feature amount by using a machine learning technology such as a DNN. In this case, the feature amount calculating unit 135 calculates an unprocessed feature amount by using the estimator 247 obtained by learning. Now, with reference to FIG. 8, an exemplary method of learning the relation between the time-series data of skeleton data and an unprocessed feature amount by using a machine learning technology is described.

FIG. 8 is an explanatory diagram illustrating an exemplary method of learning the relation between the time-series data of skeleton data and an unprocessed feature amount by using a machine learning technology. For example, the learning unit 243 may learn the relation between the time-series data of skeleton data and an unprocessed feature amount by using an Encoder-Decoder Model.

For example, in a case where posture information regarding the whole body in skeleton data in a time interval t to t+T is input, the learning unit 243 estimates an unprocessed feature amount by using a CNN (Convolutional Neural Network) as an Encoder. Further, the learning unit 243 outputs the posture of the whole body in the skeleton data in the time interval t to t+T by using the CNN as a Decorder for the estimated unprocessed feature amount.

Note that, although FIG. 8 illustrates the example in which the posture of the whole body is input as the time-series data of the skeleton data, other motion-related information such as joint positions or velocities, or multiple pieces of information may be input, for example. Further, the Encoder-Decorder Model according to the present disclosure may have a structure with more layers or a more complex structure or use another machine learning technology such as an RNN (Recurrent Neural Network).

Further, the learning unit 243 may learn the relation between the time-series data of skeleton data and an unprocessed feature amount by using Deep Metric Learning. For example, the learning unit 243 may learn the relation between the time-series data of skeleton data and an unprocessed feature amount by using Triplet Loss.

When Triplet Loss is used, data (positeve date) that is similar to a certain input (anchor) and data (negative date) that is dissimilar to an anchor may be artificially prepared, or similarity evaluation methods for time-series data may be used. Alternatively, pieces of data that are close in terms of time may be regarded as being similar, and pieces of data that are far in terms of time may be regarded as being dissimilar. Note that, examples of similarity evaluation methods for time-series data include DTW (Dynamic Time Warping).

Further, a dataset to be learned may be provided with information regarding class labels (for example, kick and punch). In a case where class label information is added to a dataset to be learned, an intermediate feature amount to be classified may be used as an unprocessed feature amount. Further, in a case where class labels are added to some data in a dataset to be learned, the dataset may be learned by using a machine learning technology with semi-supervised learning that uses an Encoder-Decoder Model and Triplet Loss in combination.

FIG. 9 is an explanatory diagram illustrating an exemplary method of calculating the unprocessed feature amount of each part according to the present disclosure.

As illustrated in FIG. 9, in a case where the parts of the whole body are divided into five parts, i.e., head (Head), body (Body), right arm (RArm), left arm (LArm), right leg (RLeg), and left leg (Lleg), the learning unit 243 may learn, for each part in skeleton data, the relation between the time-series data concerning each part in the skeleton data and the corresponding unprocessed feature amount by using a DNN.

For example, the learning unit 243 receives the posture of the body in skeleton data in the time interval t to t+T and estimates the unprocessed feature amount of the body in the skeleton data by using the DNN as an Encoder.

Then, the feature amount calculating unit 135 uses, for the calculated unprocessed feature amount of each part, the DNN as a Decorder to integrate the unprocessed feature amounts of the respective parts and thereby output the posture of the whole body in the skeleton data in the time interval t to t+T.

In the above, the specific example of the method of learning input and unprocessed feature amounts has been described. Note that, the learning unit 243 may combine the multiple unprocessed feature amount learning methods described above to learn the relation between input and an unprocessed feature amount.

<3-4. Weight Parameter>

In the present disclosure, the user performs motion data search-related operations when searching for motion data. Further, during the time period from the time when the user selects search start to the time when the user selects search end on the GUI, the feature amount calculating unit 135 calculates the feature amount of each predetermined time interval from the time-series data of skeleton data indicating the motion of the user.

Further, the feature amount calculating unit 135 calculates the unprocessed feature amount of each part in skeleton data indicating the motion of the user. For example, when the user has performed a kicking motion, the feature amount calculating unit 135 calculates not only the unprocessed feature amount of the leg that the user has raised for kicking, but also the unprocessed feature amount of each part such as the head and the arms, for example.

However, when motion data is searched for, the feature amounts of all time intervals or the feature amounts of all parts may not necessarily be important in some cases. As such, the feature amount calculating unit 135 according to the present disclosure calculates a processed feature amount by applying a weight parameter prepared for each time or each part to the unprocessed feature amount of each time or each part calculated from the time-series data concerning the motion of skeleton data.

FIG. 10 is an explanatory diagram illustrating an exemplary method of calculating processed parameters by applying weight parameters to unprocessed feature amounts. As illustrated in FIG. 10, the feature amount calculating unit 135 calculates a processed feature amount am by applying a weight parameter wm to each dimension or each time of an unprocessed feature amount bm of a single part j.

The unprocessed feature amount bm of the part j is represented by the determinant of $bm^j \in R^{M \times T}$. Here, M denotes the number of dimensions in the feature amount direction, and T denotes the number of time intervals divided into predetermined time intervals in the time direction. That is, FIG. 10 illustrates an example in which the number of dimensions M in the feature amount direction and the number of time intervals T in the time direction are five. Note that, the number of dimensions M in the feature amount direction may be one or greater. Further, the weight parameter wm and the processed feature amount am are also represented by the same number of rows and columns as the unprocessed feature amount bm.

Further, in FIG. 10, the magnitudes of values of each feature amount included in the unprocessed feature amount, each parameter included in the weight parameter, and each feature amount included in the processed feature amount are represented by the density of color. Note that, in FIG. 10, the degree of color density of each feature amount included in the unprocessed feature amount bm is represented by a unary value, and the degree of color density of each parameter included in the weight parameter wm and the degree of color density of each feature amount included in the processed feature amount am are represented by binary values, but various values can be included.

Further, in a case where there are multiple parts, other parts may be concatenated in the feature amount direction. For example, in a case where there are N parts, the weight parameter wm is represented by the determinant of $wm \in R^{(M \times N) \times T}$.

The weight parameter wm may be set by the user on the GUI or determined by use of the estimator 247 obtained by a machine learning technology. First, with reference to FIG. 11, an example in which a weight parameter is set by the user is described.

FIG. 11 is an explanatory diagram illustrating an exemplary weight parameter prepared for each time. FIG. 11 illustrates an example in which time-series data concerning the acceleration of the leg acquired by the sensor apparatus S attached to the leg of the user has been converted into time-series data concerning a velocity v of the leg.

For example, in a case where the user has performed a kicking motion, the sensor apparatus S acquires time-series data before, during, and after the kick. In a case where the kicking motion is determined to be characteristic in a motion data search, the user may set the weight parameters of the time intervals before and after the kick to small values or zero.

For example, the user may set the weight parameter wm for each time by using the operation display unit 110 included in the information processing terminal 10. For example, in a case where the hatched section illustrated in FIG. 11 is the time interval in which the user has performed a kicking motion, the user may set the weight parameter wm for acquiring the feature amount of the hatched section, for each time.

In a case where the hatched section is referred to as an "adoption section" and the sections other than the adoption section are referred to as a "non-adoption section," a weight parameter wmt for each time may be set by using Equation 1 below.

$$wm_t = 1/L \text{ (adoption section)}$$
$$wm_t = 0 \text{ (non-adoption section)}$$
$$\Sigma wm_t = 1 \cdots \text{ (Equation 1)}$$

Note that, L in Equation 1 is the time length of an adoption section.

The feature amount calculating unit 135 can calculate, as a processed feature amount, for example, the feature amount of the time interval in which the user has performed a kicking motion, by using Equation 1 with the weight parameter wmt set for each time for the unprocessed feature amount of each time.

Next, an example of calculating a processed feature amount by using a weight parameter $wm_j$ set for each part is described.

For example, in a case where motion data concerning a kicking motion is searched for, the user may set a weight parameter $wm_{Leg}$ for the leg raised for kicking to be greater than the weight parameter $wm_j$ for the other parts.

Further, the weight parameter wm may be set by the user with use of the operation display unit 110 or automatically set by the feature amount calculating unit 135. For example, in a case where it is assumed that a moving part is important, the feature amount calculating unit 135 may set the weight parameter $wm_j$ for a part with a velocity magnitude or velocity change amount equal to or greater than a predetermined value to be large, and may set the weight parameter $wm_j$ for a part with a velocity magnitude or velocity change amount less than the predetermined value to be small.

Further, the learning unit 243 may learn, in addition to the relation between the time-series data of skeleton data and an unprocessed feature amount, the relation between an unprocessed feature amount and the weight parameter wm.

FIG. 12 is an explanatory diagram illustrating an exemplary weight parameter learning method. The learning unit 243 learns the relation between the posture of each part in skeleton data and the unprocessed feature amount of each part in the time interval t to t+T by using the unprocessed feature amount calculation method described with reference to FIG. 9.

Further, the learning unit 243 may receive the posture of the whole body and the posture of each part in skeleton data in the time interval t to t+T and learn the relation between the unprocessed feature amount of each part and the weight parameter for each part by using DNN attention. Similarly, the learning unit 243 may receive the posture of the whole body and the posture of each part in skeleton data and learn the relation between the unprocessed feature amount of each time and the weight parameter for each time by using DNN attention. In this case, the feature amount calculating unit 235 determines the weight parameter for each time and the weight parameter for each part by using the estimator 247 obtained by learning.

<3-5. Similarity Evaluation>

The information processing terminal 10 transmits information regarding a processed feature amount to the server 20. Then, the similarity evaluating unit 239 included in the server 20 evaluates the similarity between the received processed feature amount and the feature amount of motion data held by the motion feature amount storing unit 225.

The similarity evaluating unit 239 may perform similarity evaluation by using, for example, mean squared error. For example, the time interval at the part j is denoted by t, the unprocessed feature amount of the dimension m is denoted by $^{query}f^j_{t,m}$, the feature amount of motion data is denoted by $^{dataset}f^j_{t,m}$, a weight parameter is denoted by $w^j_{t,m}$, and similarity is denoted by s. In this case, the similarity evaluating unit 239 evaluates the similarity between a processed feature amount and the feature amount of motion data by using Equation 2.

$$1/s = \Sigma_{j,t,m} w^j_{t,m} (^{query}f^j_{t,m} - ^{dataset}f^j_{t,m}) \quad \cdots \quad (\text{Equation 2})$$

Further, the similarity evaluating unit 239 may perform similarity evaluation by using, for example, a correlation coefficient. More specifically, the similarity evaluating unit 239 evaluates the similarity between a processed feature amount and the feature amount of motion data by using Equation 3.

$$S = \Sigma_{j,m} \{ (\Sigma^j_{t,m} {}^{query}f^j_{t,m} \times {}^{dataset}f^j_{t,m}) / (|^{query}f^j_m|_2 \times |^{dataset}f^j_m|_2) \} \quad \cdots$$

$$(\text{Equation 3})$$

Then, the server 20 transmits the motion data corresponding to the result of similarity evaluation by the similarity evaluating unit 239 to the information processing terminal 10. For example, the similarity evaluating unit 239 may calculate the similarity between a received processed feature amount and the feature amount of each of multiple pieces of motion data, and the server 20 may transmit a predetermined number of pieces of motion data as search results in order of high similarity to the information processing terminal 10.

Further, the user may perform an operation to exclude motion data with high similarity from search results. In this case, motion data determined by the similarity evaluating unit 239 as having similarity equal to or greater than a predetermined value is excluded from the search results.

<3-6. Correction>

Motion data acquired according to similarity evaluation can include the motion of the whole body of the user or the motion of parts with increased weight parameters that the user particularly needs. Meanwhile, the motion of all parts of the motion data may not necessarily match or be similar to the motion that the user needs.

Hence, the correction unit 143 may execute, for at least one part of motion data acquired as a search result, the processing of correcting the feature amount of the motion data. Now, with reference to FIG. 13, exemplary processing of correcting the feature amount of motion data is described.

FIG. 13 is an explanatory diagram illustrating exemplary processing of correcting the feature amount of motion data. In FIG. 13, skeleton data indicating the motion of the user acquired by the sensor apparatus S is illustrated as "query Q(t)," and the skeleton data of motion data acquired as a search result is illustrated as "search result R(t)."

For example, in a case where the user wants to correct the position and motion of the left arm in the search result R(t) to the position and motion in the query Q(t), the correction unit 143 may execute the processing of correcting the search result in reference to a set ratio set by the user as described above.

For example, the correction unit 143 executes, for at least one part in motion data received as a search result from the server 20, the processing of correcting the feature amount of the motion data by mixing a processed feature amount with the feature amount of the motion data. With this, the correction unit 143 acquires a corrected search result R'(t), which is the mixture of the query Q(t) and the search result R(t).

Further, the correction unit 143 may correct a part specified by the user as an object to be corrected, to have the same position as the position of the query Q(t).

For example, the correction unit 143 may execute correction processing using IK to make the position of the end part of the search result R(t) match the position of the query Q(t), with the posture of the search result R(t) as the initial value. Note that, when the position of a part is corrected, there is a possibility that the query Q(t) and the search result R(t) indicate different waist positions. Hence, for example, the correction unit 143 may execute correction processing based on the relative position from the waist.

Further, a part to be corrected may be specified by the user with use of the operation display unit 110 or automatically specified by the correction unit 143, for example.

In a case where a part to be corrected is automatically specified by the correction unit 143, for example, the correction unit 143 may determine the part to be corrected, in reference to a weight parameter prepared for each part. For example, the correction unit 143 may adopt the feature amount of the search result R(t) for a part with a weight parameter that satisfies a predetermined criterion and execute correction processing on a part with a weight parameter that does not satisfy the predetermined criterion, to make the part have the processed feature amount of the query Q(t).

Note that, even in a case where the user sets a set ratio between the processed feature amount of the query Q(t) and the feature amount of the search result R(t) on the GUI, the correction unit 143 may not necessarily execute correction processing based on the set ratio in question in some cases. For example, in a case where the balance of the whole body in motion data is lost when a part is corrected according to a set ratio, the correction unit 143 may execute the processing of correcting the feature amounts of the part and the other parts according to the positional relation between the respective parts.

In the above, the details according to the present disclosure have been described. Next, exemplary operation processing of the system according to the present disclosure is described.

<<4. Exemplary Operation>>

<4-1. Exemplary Operation of Information Processing Terminal>

FIG. 14 is an explanatory diagram illustrating exemplary motion data search-related operation processing of the information processing terminal 10 according to the present disclosure.

As illustrated in FIG. 14, the information processing terminal 10 acquires time-series data concerning the motion of an object from the sensor apparatus S (S101).

Next, the posture estimating unit 131 generates skeleton data from the acquired time-series data concerning the motion of the object (S105).

Subsequently, the posture estimating unit 131 converts the skeleton of each part in the generated skeleton data into a reference skeleton, thereby generating reference skeleton data (S109).

Then, the feature amount calculating unit 135 calculates the unprocessed feature amount of each part in the reference skeleton data from the time-series data of the reference skeleton data (S113).

Next, the feature amount calculating unit 135 calculates a processed feature amount by applying a weight parameter set for each time or each part to the unprocessed feature amount (S117).

Subsequently, the communication unit 120 transmits, under the control of the search requesting unit 139, a signal including information regarding the calculated processed feature amount to the server 20 (S121).

Then, the communication unit 120 receives a signal including information regarding motion data searched for by the server 20 according to the transmitted information regarding the processed feature amount (S125).

Next, the correction unit 143 corrects the feature amount of the motion data in reference to the set ratio between the processed feature amount and the feature amount of the acquired motion data (S129).

Subsequently, the operation display unit 110 displays the corrected motion data generated in reference to the corrected feature amount of the motion data (S133), and the information processing terminal 10 ends the motion data search-related operation processing.

Then, exemplary motion data search-related operation processing of the server 20 in S121 to S125 is described.

<4-2. Exemplary Operation of Server>

FIG. 15 is an explanatory diagram illustrating exemplary motion data search-related operation processing of the server 20 according to the present disclosure.

First, the communication unit 210 receives a processed feature amount from the information processing terminal 10 (S201).

Next, the similarity evaluating unit 239 calculates the similarity between the received processed feature amount and the feature amount of each of multiple pieces of motion data held by the motion feature amount converting unit (S205).

Subsequently, the similarity evaluating unit 239 acquires a predetermined number of pieces of motion data as search results in order of high similarity (S209).

Then, the communication unit 210 transmits the predetermined number of pieces of motion data acquired in S209 to the information processing terminal 10 as search results (S213), and the server 20 ends the motion data search-related operation processing.

In the above, the exemplary operation processing of the system according to the present disclosure has been described. Next, exemplary actions and effects according to the present disclosure are described.

<<5. Exemplary Action and Effect>>

According to the present disclosure described above, various actions and effects are obtained. For example, the feature amount calculating unit 135 calculates a processed feature amount by applying a weight parameter prepared for each part to an unprocessed feature amount calculated from time-series data concerning the motion of the user. With this, it can be possible to search for motion data by focusing on more important parts.

Further, the feature amount calculating unit 135 calculates a processed feature amount by applying a weight parameter prepared for each time to the unprocessed feature amount of each time calculated from time-series data concerning the motion of the user. This makes it possible to search for motion data by focusing on more important time intervals.

Further, since the estimator 247 obtained by a machine learning technology is used to determine weight parameters, the necessity for the user to input weight parameters manually is eliminated, so that user convenience can be improved.

Further, the information processing terminal 10 acquires a predetermined number of pieces of motion data as search results in order of high similarity between a processed feature amount calculated from the time-series data of skeleton data indicating the motion of the user and the feature amount of each of multiple pieces of motion data. With this, the user can select motion data including particularly desired motion information from among multiple pieces of presented motion data.

Further, in the embodiment according to the present disclosure, each of skeleton data indicating the motion of the user and the skeleton data of motion data is converted into reference skeleton data, and the feature amounts of the reference skeleton data are compared to each other. With this, the possibility of search errors due to differences between the skeleton of the user and the skeleton of motion data can be reduced.

Further, the correction unit 143 corrects, for at least one part, the feature amount of the motion data by mixing a processed feature amount with the feature amount of the motion data at a set ratio. With this, the motion of a part of motion data can be modified to the motion of the part that the user needs more, so that user convenience can be improved more.

<<6. Exemplary Hardware Configuration>>

In the above, the embodiment of the present disclosure has been described. The information processing described above, such as skeleton data generation and feature amount extraction, is achieved by the cooperation of software and the hardware of the information processing terminal 10 described below. Note that, the hardware configuration described below is also applicable to the server 20.

FIG. 16 is a block diagram illustrating the hardware configuration of the information processing terminal 10. The information processing terminal 10 includes a CPU (Central Processing Unit) 1001, a ROM (Read Only Memory) 1002, a RAM (Random Access Memory) 1003, and a host bus 1004. Further, the information processing terminal 10 includes a bridge 1005, an external bus 1006, an interface 1007, an input apparatus 1008, an output apparatus 1010, a storage apparatus (HDD) 1011, a drive 1012, and a communication apparatus 1015.

The CPU 1001 functions as an arithmetic processing apparatus and a control apparatus and controls the overall operation in the information processing terminal 10 according to various programs. Further, the CPU 1001 may be a microprocessor. The ROM 1002 stores programs, calculation parameters, and the like that the CPU 1001 uses. The RAM 1003 temporarily stores programs that are used in the execution of the CPU 1001 and parameters that appropriately change during the execution of the CPU 1001, for example. These are connected to each other by the host bus 1004 including a CPU bus or the like. The functions of the posture estimating unit 131 and the feature amount calculating unit 135 described with reference to FIG. 2 can be achieved by the cooperation of the CPU 1001, the ROM 1002, the RAM 1003, and the software.

The host bus 1004 is connected to the external bus 1006 such as a PCI (Peripheral Component Interconnect/Interface) bus through the bridge 1005. Note that, it is not necessarily required to configure the host bus 1004, the bridge 1005, and the external bus 1006 separately, and the functions of these may be implemented on a single bus.

The input apparatus 1008 includes input means for allowing the user to input information, an input control circuit configured to generate an input signal in response to input performed by the user and output the input signal to the CPU 1001, and the like. Examples of input means include a mouse, a keyboard, a touch panel, buttons, microphones, switches, and levers. The user of the information processing terminal 10 can input various types of data and processing operation instructions to the information processing terminal 10 by operating the input apparatus 1008.

Examples of output apparatus 1010 include such display apparatuses as a liquid crystal display apparatus, an OLED apparatus, and a lamp, and an audio output apparatus such as a speaker and a headphone. The output apparatus 1010 outputs, for example, reproduced content. Specifically, the display apparatus displays various types of information such as reproduced video data in text or images. Meanwhile, the audio output apparatus converts reproduced audio data or the like into audio and outputs the audio.

The storage apparatus 1011 is an apparatus for storing data. Examples of the storage apparatus 1011 may include a storage medium, a recording apparatus configured to record data on storage media, a reading apparatus configured to read data from a storage medium, and a deletion apparatus configured to delete data recorded on a storage medium. The storage apparatus 1011 includes, for example, an HDD (Hard Disk Drive). The storage apparatus 1011 in this case drives the hard disk to store programs that the CPU 1001 executes and various types of data.

The drive 1012 is a storage medium reader/writer, and is a built-in or external component of the information processing terminal 10. The drive 1012 reads information recorded on an installed removable storage medium 30, such as a magnetic disk, an optical disc, a magneto-optical disk, or a semiconductor memory, and outputs the information to the RAM 1003. Further, the drive 1012 can also write information to the removable storage medium 30.

The communication apparatus 1015 is, for example, a communication interface including a communication device or the like for connection to the network 12. Further, the communication apparatus 1015 may be a wireless LAN-compatible communication apparatus, an LTE (Long Term Evolution)-compatible communication apparatus, or a wired communication apparatus for wired communication.

<< 7. Supplementary Note>>

In the above, the preferred embodiment of the present disclosure has been described in detail with reference to the accompanying drawings, but the present disclosure is not limited to this example. It is apparent that various changes or modifications could be arrived at by persons who have ordinary knowledge in the technical field to which the present disclosure belongs, within the scope of the technical ideas described in the appended claims, and it is therefore understood that such changes or modifications naturally belong to the technical scope of the present disclosure.

For example, the information processing terminal 10 may further have all or some functional configurations of the server 20 according to the present disclosure. In a case where the information processing terminal 10 has all functional configurations of the server 20 according to the present disclosure, the information processing terminal 10 can execute the series of search-related processing processes without communication via the network 1. Further, in a case where the information processing terminal 10 has some functional configurations of the server 20 according to the present disclosure, for example, the information processing terminal 10 may receive multiple pieces of motion data from the server 20 in advance by using communication via the network 1. Further, the information processing terminal 10 may evaluate the similarity between a processed feature amount calculated by the feature amount calculating unit 135 and the multiple pieces of motion data received from the server 20 in advance, and may search for motion data according to the results of similarity evaluation.

The respective steps of the processing of the information processing terminal 10 and the server 20 herein are not necessarily required to be performed in chronological order in the order described as the flowcharts. For example, the respective steps of the processing of the information processing terminal 10 and the server 20 may be performed in orders different from the orders described as the flowcharts.

Further, it is also possible to create a computer program for causing the hardware built in the information processing terminal 10, such as the CPU, the ROM, and the RAM, to exhibit functions equivalent to those of the respective configurations of the information processing terminal 10 described above. Further, a storage medium having stored therein the computer program in question is also provided.

Further, the effects described herein are merely illustrative and exemplary and are not limited. That is, the technology according to the present disclosure may provide other effects that are apparent for persons skilled in the art from the description of the present specification, in addition to the above-mentioned effects or in place of the above-mentioned effects.

Note that, the following configurations also belong to the technical scope of the present disclosure.

(1) An information processing apparatus including:

an acquisition unit configured to acquire a processed feature amount that is a feature amount calculated by applying, to an unprocessed feature amount that is a feature amount of each time or each part of an object calculated from time-series data concerning motion of the object, a weight parameter prepared for each time or each part; and

a search unit configured to search for motion data by using the processed feature amount acquired by the acquisition unit.

(2) The information processing apparatus according to (1) above, in which the weight parameter that is applied to the unprocessed feature amount is determined by an estimator obtained by learning a relation between the feature amount of each part and the weight parameter for each part.

(3) The information processing apparatus according to (1) or (2) above, in which the weight parameter that is to be applied to the unprocessed feature amount is determined by an estimator obtained by learning a relation between the feature amount of each time and the weight parameter for each time.

(4) The information processing apparatus according to any one of (1) through (3) above, in which the search unit calculates similarity between the processed feature amount of the object acquired by the acquisition unit and a feature amount of each of multiple pieces of motion data, and searches for motion data in reference to a result of similarity calculation.

(5) The information processing apparatus according to (4) above, in which the search unit acquires, in reference to the result of similarity calculation, a predetermined number of pieces of motion data as search results in order of high feature amount similarity with the processed feature amount.

(6) The information processing apparatus according to (4) or (5) above, in which the acquisition unit searches for the motion data by comparing a processed feature amount calculated from time-series data concerning motion of a reference object obtained by converting a skeleton of the object into a reference skeleton to a feature amount calculated from reference motion data obtained by converting a skeleton of skeleton data into the reference skeleton.

(7) The information processing apparatus according to any one of (1) through (6) above, further including:

a correction unit configured to correct, for at least one part, a feature amount of the motion data by mixing the processed feature amount with the feature amount of the motion data at a set ratio.

(8) The information processing apparatus according to any one of (1) through (7) above, in which the feature amount of each part of the object includes at least one of velocity, position, or posture.

(9) An information processing method that is executed by a computer, the information processing method including:

acquiring a processed feature amount that is a feature amount calculated by applying, to an unprocessed feature amount that is a feature amount of each time or each part of an object calculated from time-series data concerning motion of the object, a weight parameter prepared for each time or each part; and

searching for motion data by using the processed feature amount acquired.

(10) A program for causing a computer to achieve:

an acquisition function of acquiring a processed feature amount that is a feature amount calculated by applying, to an unprocessed feature amount that is a feature amount of each time or each part of an object calculated from time-series data concerning motion of the object, a weight parameter prepared for each time or each part; and

a search function of searching for motion data by using the processed feature amount acquired by the acquisition function.

[Reference Signs List]

10: Information processing terminal
20: Server
110: Operation display unit
120: Communication unit
130: Control unit
131: Posture estimating unit
135: Feature amount calculating unit
139: Search requesting unit
143: Correction unit

210: Communication unit
220: Storage unit
221: Motion data storing unit
225: Motion feature amount storing unit
230: Control unit
231: Reference skeleton converting unit
235: Feature amount calculating unit
239: Similarity evaluating unit
243: Learning unit
247: Estimator

**Claims**

1. An information processing apparatus comprising:

   an acquisition unit configured to acquire a processed feature amount that is a feature amount calculated by applying, to an unprocessed feature amount that is a feature amount of each time or each part of an object calculated from time-series data concerning motion of the object, a weight parameter prepared for each time or each part; and
   a search unit configured to search for motion data by using the processed feature amount acquired by the acquisition unit.

2. The information processing apparatus according to claim 1, wherein the weight parameter that is to be applied to the unprocessed feature amount is determined by an estimator obtained by learning a relation between the feature amount of each part and the weight parameter for each part.

3. The information processing apparatus according to claim 2, wherein the weight parameter that is to be applied to the unprocessed feature amount is determined by an estimator obtained by learning a relation between the feature amount of each time and the weight parameter for each time.

4. The information processing apparatus according to claim 3, wherein the search unit calculates similarity between the processed feature amount of the object acquired by the acquisition unit and a feature amount of each of multiple pieces of motion data, and searches for motion data in reference to a result of similarity calculation.

5. The information processing apparatus according to claim 4, wherein the search unit acquires, in reference to the result of similarity calculation, a predetermined number of pieces of motion data as search results in order of high feature amount similarity with the processed feature amount.

6. The information processing apparatus according to claim 5, wherein the acquisition unit searches for the motion data by comparing a processed feature amount calculated from time-series data concerning motion of a reference object obtained by converting a skeleton of the object into a reference skeleton to a feature amount calculated from reference motion data obtained by converting a skeleton of skeleton data into the reference skeleton.

7. The information processing apparatus according to claim 6, further comprising:
   a correction unit configured to correct, for at least one part, a feature amount of the motion data by mixing the processed feature amount with the feature amount of the motion data at a set ratio.

8. The information processing apparatus according to claim 7, wherein the feature amount of each part of the object includes at least one of velocity, position, or posture.

9. An information processing method that is executed by a computer, the information processing method comprising:

   acquiring a processed feature amount that is a feature amount calculated by applying, to an unprocessed feature amount that is a feature amount of each time or each part of an object calculated from time-series data concerning motion of the object, a weight parameter prepared for each time or each part; and
   searching for motion data by using the processed feature amount acquired.

17

10. A program for causing a computer to achieve:

an acquisition function of acquiring a processed feature amount that is a feature amount calculated by applying, to an unprocessed feature amount that is a feature amount of each time or each part of an object calculated from time-series data concerning motion of the object, a weight parameter prepared for each time or each part; and
a search function of searching for motion data by using the processed feature amount acquired by the acquisition function.

# FIG.1

# F I G . 2

10

INFORMATION PROCESSING TERMINAL

110
OPERATION DISPLAY UNIT

120
COMMUNICATION UNIT

130
CONTROL UNIT

POSTURE ESTIMATING UNIT — 131

↓

FEATURE AMOUNT CALCULATING UNIT — 135

↓

SEARCH REQUESTING UNIT — 139

↓

CORRECTION UNIT — 143

# FIG.3

20

SERVER

220
STORAGE UNIT

221
MOTION DATA
STORING UNIT

225
MOTION FEATURE
AMOUNT
STORING UNIT

230
CONTROL UNIT

231
REFERENCE SKELETON
CONVERTING
UNIT

235
FEATURE AMOUNT
CALCULATING
UNIT

210
COMMUNICATION
UNIT

239
SIMILARITY
EVALUATING
UNIT

243
LEARNING UNIT

247
ESTIMATOR

# FIG.4

# FIG.5

# FIG.6

# FIG.7

PD

SD

S5

SP7
SB1

SP1

S2

S1

S5

SP2

S3

S4

# FIG.8

POSTURE
(WHOLE BODY)
t~t+T
→
Encoder
(CNN)
→
FEATURE AMOUNT
→
Decoder
(CNN)
→
POSTURE
(WHOLE BODY)
t~t+T

# FIG.9

# FIG.10

# FIG.11

FIG.12

EP 4 261 709 A1

⊗ Elementwise Product

POSTURE (HEAD)
t~t+T

Head
Enc

Head
feat

$w^{head}$

POSTURE (LEFT LEG)
t~t+T

LLeg
Enc

LLeg
feat

$w^{lleg}$

POSTURE (WHOLE BODY)
t~t+T

Attention
Enc

w

Dec

POSTURE
(WHOLE BODY)
t~t+T

# FIG.13

# FIG.14

```
                    ┌─────────────┐
                    │    START    │
                    └──────┬──────┘
                           │
                           ▼
        ┌──────────────────────────────────┐
        │    ACQUIRE TIME-SERIES DATA       │──── S101
        │  CONCERNING MOTION OF OBJECT      │
        └──────────────────┬───────────────┘
                           │
                           ▼
        ┌──────────────────────────────────┐
        │     GENERATE SKELETON DATA        │──── S105
        │       CONCERNING OBJECT           │
        └──────────────────┬───────────────┘
                           │
                           ▼
        ┌──────────────────────────────────┐
        │  CONVERT SKELETON DATA CONCERNING │──── S109
        │ OBJECT INTO REFERENCE SKELETON DATA│
        └──────────────────┬───────────────┘
                           │
                           ▼
        ┌──────────────────────────────────┐
        │   CALCULATE UNPROCESSED FEATURE   │
        │   AMOUNT FROM TIME-SERIES DATA    │──── S113
        │     OF REFERENCE SKELETON DATA    │
        └──────────────────┬───────────────┘
                           │
                           ▼
        ┌──────────────────────────────────┐
        │    CALCULATE PROCESSED FEATURE    │──── S117
        │ AMOUNT BY USING WEIGHT PARAMETER  │
        └──────────────────┬───────────────┘
                           │
                           ▼
        ┌──────────────────────────────────┐
        │       TRANSMIT PROCESSED          │──── S121
        │    FEATURE AMOUNT TO SERVER       │
        └──────────────────┬───────────────┘
                           │
                           ▼
        ┌──────────────────────────────────┐
        │  ACQUIRE MOTION DATA SEARCHED FOR │──── S125
        │ ACCORDING TO PROCESSED FEATURE AMOUNT│
        └──────────────────┬───────────────┘
                           │
                           ▼
        ┌──────────────────────────────────┐
        │ CORRECT FEATURE AMOUNT OF MOTION  │──── S129
        │   DATA IN REFERENCE TO SET RATIO  │
        └──────────────────┬───────────────┘
                           │
                           ▼
        ┌──────────────────────────────────┐
        │   DISPLAY CORRECTED MOTION DATA   │──── S133
        └──────────────────┬───────────────┘
                           │
                           ▼
                    ┌─────────────┐
                    │     END     │
                    └─────────────┘
```

29

# FIG.15

```
┌─────────┐
│  START  │
└─────────┘
     │
     ▼
┌────────────────────────────────────┐
│ RECEIVE PROCESSED FEATURE AMOUNT    │──S201
└────────────────────────────────────┘
     │
     ▼
┌────────────────────────────────────┐
│ CALCULATE SIMILARITY BETWEEN        │
│ PROCESSED FEATURE AMOUNT AND        │──S205
│ FEATURE AMOUNT OF EACH OF MULTIPLE  │
│ PIECES OF MOTION DATA               │
└────────────────────────────────────┘
     │
     ▼
┌────────────────────────────────────┐
│ ACQUIRE PREDETERMINED NUMBER OF     │
│ PIECES OF MOTION DATA AS SEARCH     │
│ RESULTS IN ORDER OF HIGH SIMILARITY │──S209
│ OF FEATURE AMOUNTS IN REFERENCE TO  │
│ RESULTS OF SIMILARITY CALCULATIONS  │
└────────────────────────────────────┘
     │
     ▼
┌────────────────────────────────────┐
│ TRANSMIT ACQUIRED SEARCH RESULTS    │──S213
│ TO INFORMATION PROCESSING TERMINAL  │
└────────────────────────────────────┘
     │
     ▼
┌─────────┐
│   END   │
└─────────┘
```

# FIG.16

INFORMATION PROCESSING TERMINAL 10

| | | |
|---|---|---|
| CPU 1001 | ROM 1002 | RAM 1003 |

1004

BRIDGE 1005

1006

1007

INTERFACE

| INPUT APPARATUS 1008 | OUTPUT APPARATUS 1010 | STORAGE APPARATUS (HDD) 1011 | DRIVE 1012 | COMMUNICATION APPARATUS 1015 |
|---|---|---|---|---|

REMOVABLE STORAGE MEDIUM 30

1

## INTERNATIONAL SEARCH REPORT

| International application No. |
|---|
| PCT/JP2021/006290 |

**A. CLASSIFICATION OF SUBJECT MATTER**
Int. Cl. G06F16/732(2019.01)i, A61B5/11(2006.01)i, G06F16/783(2019.01)i, G06T13/40(2011.01)i
FI: G06F16/732, G06F16/783, G06T13/40, A61B5/11 230
According to International Patent Classification (IPC) or to both national classification and IPC

**B. FIELDS SEARCHED**

Minimum documentation searched (classification system followed by classification symbols)
Int. Cl. G06F16/00-16/958, A61B5/11, G06T13/40

Documentation searched other than minimum documentation to the extent that such documents are included in the fields searched
Published examined utility model applications of Japan    1922-1996
Published unexamined utility model applications of Japan  1971-2021
Registered utility model specifications of Japan          1996-2021
Published registered utility model applications of Japan  1994-2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2010-33163 A (KDDI CORP.) 12 February 2010 | 1-2, 9-10 |
| A | (2010-02-12), paragraphs [0004]-[0006] | 3-8 |
| X | 高原健輔ほか．特徴ベクトルの簡略離散表現によるモーション検索高速化 | 1-2, 9-10 |
| A | 手法．インタラクション 2015 論文集．[online], 26 February 2015, pp. 390-395 [retrieved on 30 June 2017], Internet: <URL: http://www.interaction-ipsj.org/proceedings/2015/data/20150226/A62.pdf>, in particular, "3. Proposed Method", (TAKAHARA, Kensuke et al. A Rapid Motion Retrieval Technique using Simple and Discrete Representation of Feature Vector. IPSJ Interaction 2015.) | 3-8 |

☒ Further documents are listed in the continuation of Box C.　　☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 20.04.2021 | 27.04.2021 |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

| International application No. |
| --- |
| PCT/JP2021/006290 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | XIAO, Qinkun et al. Human Motion Retrieval Based on Deep Learning and Dynamic Time Warping, 2017 2nd International Conference on Robotics and Automation Engineering (IGRAE), 31 December 2017, pp. 426-430, entire text, all drawings | 1-10 |

Form PCT/ISA/210 (continuation of second sheet) (January 2015)

**INTERNATIONAL SEARCH REPORT**
Information on patent family members

International application No.

PCT/JP2021/006290

| Patent Documents referred to in the Report | Publication Date | Patent Family | Publication Date |
|---|---|---|---|
| JP 2010-33163 A | 12.02.2010 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20120038628